# EUROPÄISCHE PATENTANMELDUNG

(11) **EP 2 380 517 A1**
(43) Veröffentlichungstag der Anmeldung: **26.10.2011**
(21) Anmeldenummer: 11160806.3
(22) Anmeldetag: 01.04.2011
(51) Int. Cl.: A61B 18/14

(54) **Elektrode für einen elektrophysiologischen Ablationskatheter**

(30) Priorität: 20.04.2010 US 325825 P
(71) Anmelder: VascoMed GmbH, 79589 Binzen (DE)
(72) Erfinder: Geistert, Wolfgang, 79618, Rheinfelden (DE); Erben, Martin, 13507, Berlin (DE); Kiefer, Andreas, 79541, Lörrach (DE); Kaufmann, Ralf, 79540, Lörrach (DE)
(74) Vertreter: Lindner-Vogt, Karin L.

(57) **Zusammenfassung**

Die Erfindung betrifft eine Elektrode für einen elektrophysiologischen Ablationskatheter mit einem sich entlang einer Längsachse erstreckenden Elektrodenkörper, wobei der Elektrodenkörper eine Elektrodenaußenfläche zur Abgabe von hochfrequenten Signalen und/oder zur Messung von physiologischen Signalen, eine erste Anbringungsstelle an einem ersten Ende, an welchem die Elektrode an einem ersten Katheterschaft angebracht ist, ein Spülmittellumen in Erstreckung parallel zur Längsachse, durch welches Spülmittel aus dem ersten Katheterschaft in die Elektrode geleitet werden kann und welches am ersten Ende des Elektrodenkörpers eine Öffnung bildet, welche in Verbindung mit einem Lumen des ersten Katheterschaftes steht, und mindestens einen in Verbindung mit dem Spülmittellumen stehenden Spülmitteldurchgang umfasst, der in einem Winkel zur Längsachse angeordnet ist, wobei der Spülmitteldurchgang an der Elektrodenaußenfläche eine erste und eine zweite Öffnung bildet, durch die das Spülmittel in die Umgebung als Spülmittelstrom abgegeben werden kann.

## Beschreibung

Vorhofflimmern ist die häufigste Herzrhythmusstörung, an der allein in Deutschland rund eine Million vor allem ältere Menschen leiden. Experten schätzen, dass die Zahl der Betroffenen bis zum Jahr 2050 auf 2,5 Millionen steigen wird. Ursache des abnormen Herzrhythmus können allgemeine Gesundheitsprobleme oder Herzleiden sein - aber auch Stress, Alkohol, Koffein, schwere Infektionen oder Medikamente.
Vorhofflimmern bedeutet, dass die Vorhöfe des Herzens unregelmäßig und mit einer Frequenz von mehr als 300 Schlägen pro Minute arbeiten. Es entsteht, wenn die elektrischen Signale nicht vom Sinusknoten, dem herzeigenen Schrittmacher, ausgehen, sondern zusätzlich von anderen Ursprungsorten (Foci), die meistens in den Pulmonalvenen ihren Ursprung haben. Dadurch werden kreisende elektrische Erregungen in den Vorhöfen ausgelöst. Meist beschränkt der Atrioventrikularknoten (AV-Knoten), der die Signale ausgehend vom Sinusknoten über die Vorhöfe zu den Herzkammern (Ventrikel) übermittelt, größtenteils die Anzahl der Impulse, so dass normalerweise nicht das ganze Herz mit dieser schnellen Frequenz schlägt. Dennoch hat der Herzmuskel wegen der fehlgesteuerten Erregung nicht genügend Zeit, sich ausreichend zusammenzuziehen, um den nächsten Pumpvorgang einzuleiten. Die Folge davon ist, dass weniger Blut und somit Sauerstoff von den Vorhöfen in die Herzkammern und von hier in den Körperkreislauf gelangt. Die gesunkene Pumpleistung des Herzens führt bei ca. 80 Prozent der Patienten zur Einschränkung der körperlichen Leistungsfähigkeit durch Herzklopfen, Kurzatmigkeit, Schwindel oder Angst, und der Lebensqualität. Bei anhaltendem Vorhofflimmern besteht ein erhöhtes Risiko für Schlaganfall, da sich durch die geringere Pumpleistung des Herzens Blutgerinnsel im linken Vorhof bilden und in das Gehirn gelangen können.

Ein Therapiekonzept, das Vorhofflimmern dauerhaft heilen kann, ist die Ablation. Hierbei wird der Bereich des Herzens, der die Rhythmusstörung hervorruft oder fördert, durch Energieabgabe thermisch verödet (abladiert). Die Ablation zerstört den oder die Ursprungsherde und isoliert das überleitende Herzgewebe durch Barrieren aus Narbengewebe, das nicht mehr elektrisch leitfähig ist. Weil die abnormen elektrischen Signale nun nicht mehr in den Vorhof gelangen, bestimmt allein, wie von der Natur vorgesehen, der Sinusknoten die Erregung - der natürliche Herzrhythmus ist wieder hergestellt. Viele Episoden von Vorhofflimmern werden nicht nur durch einzelne Punkte, sondern durch mehrere Ursprungsorte ausgelöst. Die Ärzte isolieren diese Ursprungsorte durch Ablationslinien, die die Vorhöfe in miteinander verbundene Korridore und Sackgassen unterteilen, so dass die elektrischen Impulse nur noch den vorgegebenen Leitungsbahnen folgen. Für die Ablation stehen verschiedene Methoden mit unterschiedlichen Energieformen zur Verfügung. Zu den am häufigsten verwendeten gehören Hochfrequenzstrom mittels minimalinvasiver Katheterablation mit einem elektrophysiologischen Katheter.

Bei der minimalinvasiven Katheterablation durch einen Arzt, insbesondere einen Elektrophysiologen, wird ein elektrophysiologischer Ablationskatheter über eine Vene - meist im Leistenbereich - eingeführt und bis zum Herzen vorgeschoben. Anschließend wird die Katheterspitze in direkten Kontakt mit dem Herzgewebe gebracht und gibt eine hochfrequente Energie abgegeben, um eine Verödung des Herzgewebes zu erreichen. Wesentlich erleichtert wird die Planung und Durchführung von Katheterablationen durch das so genannte Mapping. Diese elektrophysiologische Untersuchung bildet die Erregungsleitung im Vorhof dreidimensional ab und ermöglicht so eine punktgenaue Navigation des elektrophysiologischen Ablationskatheters. Zudem reduziert sie die durch konventionelle Röntgen-Durchleuchtung verursachte Strahlenbelastung. Die Ablation kann das Vorhofflimmern zu einem hohen Prozentsatz vollständig beseitigen, so dass die Patienten nach einer gewissen Zeit weitgehend beschwerdefrei sind. Vorteilhaft ist, dass bei der Ablation mittels eines elektrophysiologischen Ablationskatheters in Einzelfällen zwar manchmal länger dauert, dem Patienten aber eine belastende chirurgische Operation erspart.

Ein solcher elektrophysiologischer Ablationskatheter besteht in der Regel aus einem lang gestreckten Katheterschaft, welcher mehrere Lumen aufweist, unter anderem meist ein Lumen für Steuermittel wie Zugdrähte und ein Lumen, in welchem isoliert geführte Signalleitungen zur Messung von Körpersignalen und/oder zur Überleitung von hochfrequenten Signalen zur Erzeugung von Ablationsenergie zu Elektroden am distalen Ende geführt werden. Bei den Elektroden kann es sich um eine oder mehrere Ringelektroden am Katheterschaft handeln oder eine am distalen Ende des Katheterschaftes befindliche Kopfelektrode. Das dem distalen Ende gegenüber liegenden proximalen Ende wird nicht in den Körper eingeführt und umfasst meist Steuermittel zur aktiven Steuerung des distalen Katheterschaftendes durch den Elektrophysiologen und Anschlüsse zur reversiblen Verbindung mit Messeinrichtungen, Hochfrequenz- (HF-) Generatoren und Kühlmittelpumpen oder Kombinationen daraus. Der Katheterschaft kann dabei verschiedene Abschnitte aus unterschiedlichem Material und/oder Härten aufweisen, welche Vorteile bei der Steuerbarkeit bieten. Exemplarisch ist ein solcher Katheter in der US RE 34,502 dargestellt.
In einer Ausführung weist ein solcher Katheter auch die Möglichkeit zur Kühlung auf. In einer Variante, ausgeführt als geschlossene Kühlung, weist die Kopfelektrode einen Innenraum auf, in den über ein zusätzliches Lumen im Katheterschaft Spülmittel vom proximalen Ende her geführt wird. Dieses Medium wird zur Abfuhr der Wärme über ein weiteres Lumen im Katheterschaft wieder zurück zum proximalen Ende geführt und entsorgt.
Eine andere Variante ist als so genannte offene Kühlung ausgelegt und werden auch Irrigationskatheter genannt. In dieser Variante weist die Kopfelektrode am distalen Ende des Katheterschaftes Öffnungen auf, durch welche das durch das Spülmittellumen geführte Spülmittel austreten kann.

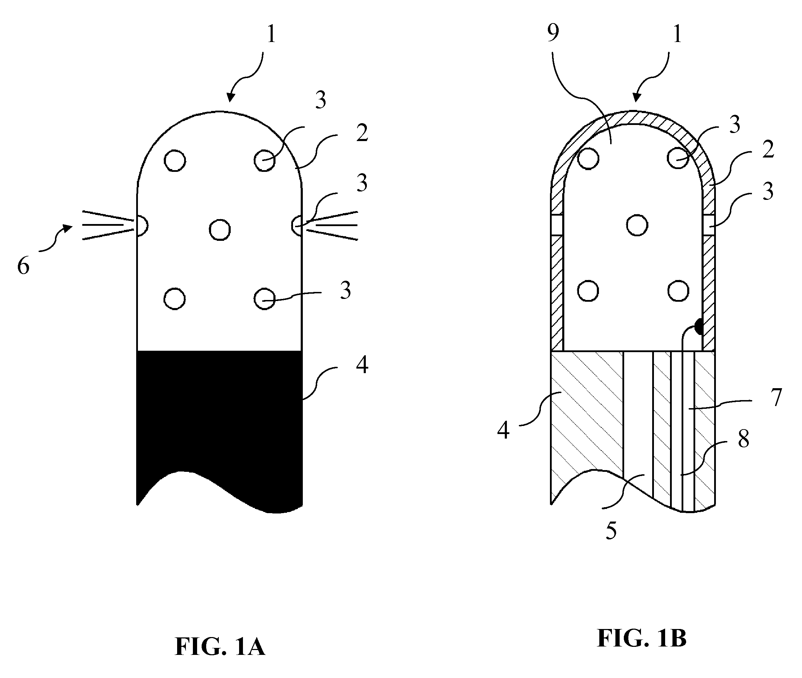

Fig. 1A und Fig. 1B zeigen eine erste Ausführungsform einer solchen bekannten Kopfelektrode mit Öffnungen (so genannte Irrigationsöffnungen) für einen elektrophysiologischen Ablationskatheters mit offener Kühlung. Die Kopfelektrode 1 ist durch eine Metallhülle 2 gebildet, in welcher Öffnungen 3 vorgesehen sind. Die Metallhülle umschließt dabei einen Innenraum 9. Die Öffnungen 3 können beispielsweise durch spangebende Verfahren oder andere Verfahren wie Lasern hergestellt werden. Die Kopfelektrode 1 ist am distalen Ende eines lang gestreckten, zur Einführung in ein Körperlumen geeigneten Katheterschaftschaftes 4 befestigt. Der Katheterschaft 4 umfasst dabei mehrere Lumen wie beispielsweise Lumen für Steuerdrähte oder ein Lumen 7 für isoliert geführte Signalleitungen zur Messung von Körpersignalen und/oder zur Überleitung von hochfrequenten Signalen zur Erzeugung von Ablationsenergie an der Kopfelektrode. Weiterhin umfasst der Katheterschaft 4 ein Spüllumen 5, durch welches ein Spülmittel wie beispielsweise physiologische Kochsalzlösung zur Kopfelektrode 1 geführt wird. Das Spülmittel wird dabei mit einem solchen Druck und in einer solchen Menge kontinuierlich zur Verfügung gestellt, dass es den Innenraum der Kopfelektrode ausfüllt und durch die Öffnungen 3 aus dem Innenraum 9 an die Umgebung abgegeben wird, um so eine Kühlung beispielsweise des Herzgewebes zu gewährleisten. Zur Sicherstellung des Spülmitteldruckes und der Spülmittelmenge ist der elektrophysiologische Katheter an seinem proximalen, der Kopfelektrode abgewandten Seite unter Anderem mit einer Kühlmittelpumpe verbunden. Eine solche Pumpe und ein Generator zur Erzeugung der hochfrequenten Energie ist beispielsweise der US 2009/0187186 zu entnehmen, welche in diese Patentanmeldung vollumfänglich einbezogen ist. Bei Austritt aus den Öffnungen 3 bildet das Spülmittel einen Spülstrom 6, der im Wesentlichen orthogonal zur die Öffnung umgebende äußeren Kopfelektrodenfläche gerichtet ist. Nicht gezeigt ist ein optionaler Temperatursensor, welcher im Lumen 7 oder in einem weiteren Lumen geführt sein kann. Durch die nahezu komplette Ausfüllung mit Spülmittel erzeugt diese Kopfelektrodenkonfiguration gute Kühleigenschaften in der eigentlichen Elektrode. Jedoch ist der Kühleffekt auf das umgebende Gewebe unzureichend, da die orthogonal zur Kopfelektrodenfläche austretende Spielströme nicht in der Lage sind, alle Außenflächen der Kopfelektrode ausreichend zu kühlen. Dies gilt insbesondere für die Übergangsbereiche zwischen Katheterschaft 4 und Kopfelektrode 1. In diesen Übergangsbereichen herrscht bei HF-Ablationsenergieabgabe aufgrund des Kanteneffekts eine erhöhte Stromdichte, die mit einer erhöhten Wärmeerzeugung verbunden ist. Dadurch ist in diesen Übergangbereichen die Gefahr der unerwünschten Koagelbildung besonders hoch.

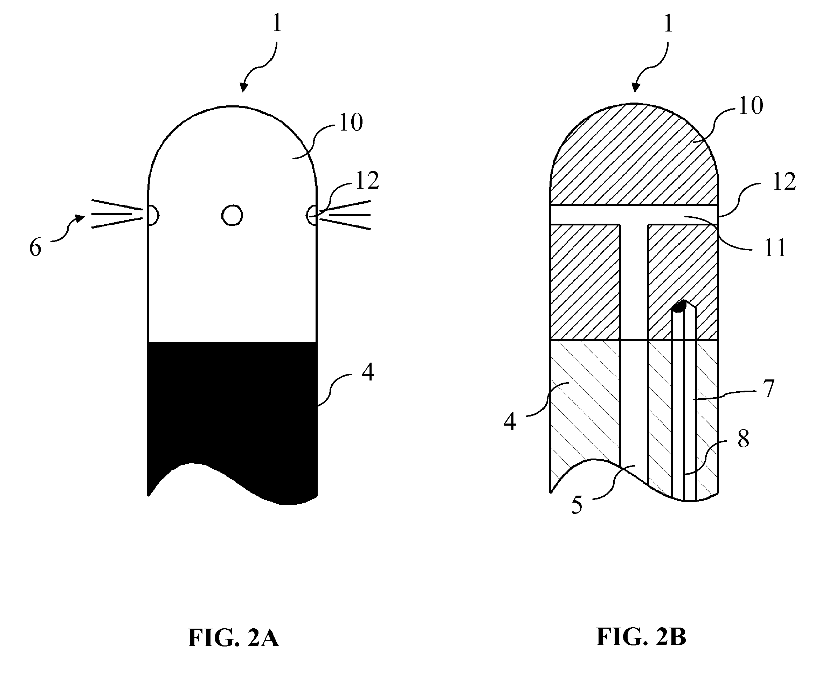

Fig. 2A und Fig. 2B zeigen eine weitere aus dem Stand der Technik bekannte Kopfelektrode für einen elektrophysiologischen Katheter mit gleichem Schaftaufbau wie oben beschrieben. Die Kopfelektrode 1 jedoch ist durch ein massives Metallelement 10 gebildet, in welchem Durchgänge 11 mit Öffnungen 12 vorgesehen sind, durch welche das Spülmittel an die Katheterumgebung austreten kann. Die Durchgänge, vorzugsweise sechs an Zahl, schneiden das Spüllumen 5 orthogonal ans dessen Ende. Das Spülmittel wird dabei in einem solchen Druck und einer solchen Menge kontinuierlich zur Verfügung gestellt, dass es die Öffnungen 12 der Durchgänge 11 unter einem bestimmten Druck verlässt und dabei einen Spülstrom 6 erzeugt, um so eine Kühlung beispielsweise des Herzgewebes zu gewährleisten. Auch in dieser bekannten Ausführungsform ist der Spülstrom 6 ist im Wesentlichen orthogonal zur die Öffnung 12 umgebenden äußeren Kopfelektrodenfläche gerichtet. Auch diese Ausführung hat das Problem, dass durch die Orthogonalität des Spülstromes in Bezug auf die äußere Kopfelektrodenoberfläche der Kühleffekt auf das umgebende Gewebe unzureichend ist. Dies gilt insbesondere für die Grenzbereiche zwischen Katheterschaft 4 und Kopfelektrode 1.

Es ist daher Aufgabe der vorliegenden Erfindung, die Kühlung der Elektroden, insbesondere der Kopfelektrode eines elektrophysiologischen Ablationskatheter effektiver zu gestalten und Koagulationen des Körpermediums in Umgebung der Elektroden zu vermeiden. Diese Aufgabe wird durch eine Elektrode nach Anspruch 1 und einem elektrophysiologischen Ablationskatheter nach Anspruch 7 gelöst.

Der Erfindung liegt die Erkenntnis zu Grunde, dass die aus dem Stand der Technik bekannten Irrigationslösungen mit Winkeln von etwa 90 Grad zur Oberfläche der Elektrode nicht ausreichend sind, um eine komplette und effektive Abdeckung der Elektrodenaußenfläche mit Spülmittel und damit eine effektive Kühlung während der Abgabe des hochfrequenten Ablationssignales zu gewährleisten. Insbesondere hat es sich herausgestellt, dass die im Stand der Technik zur Verfügung stehenden Lösungen nicht in der Lage sind, den Übergang zwischen Katheterschaftmaterial und Elektrodenmaterial so abzudecken, dass eine Kühlung explizit dieses Katheterabschnittes gewährleistet und das vermehrte Auftreten von Koagulationen verhindert wird.

Die Erfindung betrifft daher eine Elektrode für einen elektrophysiologischen Ablationskatheter mit einem sich entlang einer Längsachse erstreckenden Elektrodenkörper, wobei der Elektrodenkörper eine Elektrodenaußenfläche zur Abgabe von hochfrequenten Signalen und/oder zur Messung von physiologischen Signalen, eine erste Anbringungsstelle an einem ersten Ende, an welchem die Elektrode an einem ersten Katheterschaft angebracht ist, ein Spülmittellumen in Erstreckung parallel zur Längsachse, durch welches Spülmittel aus dem ersten Katheterschaft in die Elektrode geleitet werden kann und welches am ersten Ende des Elektrodenkörpers eine Öffnung bildet, welche in Verbindung mit einem Lumen des ersten Katheterschaftes steht, und mindestens einen in Verbindung mit dem Spülmittellumen stehenden Spülmitteldurchgang umfasst, der in einem Winkel zur Längsachse angeordnet ist, wobei der Spülmitteldurchgang an der Elektrodenaußenfläche eine erste und eine zweite Öffnung bildet, durch die das Spülmittel in die Umgebung als Spülmittelstrom abgegeben werden kann.
Die Erfindung zeichnet sich insbesondere dadurch aus, dass der Winkel zur Längsachse ein solches Gradmaß aufweist, dass der Spülmitteldurchgang eine Erstreckungskomponente parallel zur Längsachse der Elektrode aufweist, so dass der aus der mindestens einen Öffnungen austretende Spülmittelstrom eine Erstreckungskomponente entlang der Elektrodenaußenflächen aufweist und sich so verteilt, dass die Elektrode und deren unmittelbare Umgebung gekühlt ist. Das bedeutet, dass der Spülmittelstrom, der beim Austritt des Spülmittels aus den Öffnungen der Spülmitteldurchgänge eine Richtung entlang Erstreckungskomponente in Richtung der Längsachse erhält, die sich über die Umgrenzung der Elektrode hinaus erstreckt, das heißt, in Richtung des ersten Katheterschaftes von der Elektrode weg in Richtung des zu ablatierenden Gewebes und vor Allem über die Anbringungsstelle am ersten Ende des Elektrodenkörpers hinweg zum ersten Katheterschaft, an dem es bevorzugt zu Koagulationen kommen kann. Somit kann eine besonders gute Abdeckung mit Spülmittel erfolgen.

In einer besonderen Ausführungsform verläuft der Spülmitteldurchgang vollständig durch die Elektrode hindurch, so dass der Spülmitteldurchganges an der diametral entgegen gesetzt zur ersten Öffnung befindlichen Elektrodenaußenfläche eine zweite Öffnung aufweist.

Zur Versorgung der Spülmitteldurchgänge bildet das Spülmittellumen am ersten Ende des Elektrodenkörpers eine Öffnung, welches in Verbindung mit einem Lumen des ersten Katheterschaftes steht. Die Elektrodenaußenfläche im Bereich der ersten und/oder zweiten Öffnung kann dabei trichterförmige Einbuchtungen oder eine radial umlaufende Nut aufweisen, um die Verteilung des Spülmediums entlang der Anbringungsstelle zum ersten Katheterschaft zu gewährleisten.

Der mindestens eine Spülmitteldurchgang durchläuft den rotationssymmetrischen Elektrodenkörper diagonal so, dass das Spülmittellumen und der mindestens eine Spülmitteldurchgang in Verbindung stehen. Diagonal bedeutet in diesem Sinne, dass die mindestens einen Spülmitteldurchgänge bei Blickrichtung vom ersten Ende des Elektrodenkörpers den kreisförmigen Zylindergrundriss des Elektrodenkörpers als Sekante durchschneiden können. In diesem Fall befindet sich das Spülmittellumen parallel zur Längsachse des Elektrodenkörpers. Befindet sich das Spülmittellumen jedoch auf der Längsachse, so bilden die Spülmitteldurchgänge eine Diametrale, welche ebenfalls unter dem Begriff "diagonal" oder "Diagonale" zusammengefasst ist, damit eine Verbindung zum Spülmittellumen hergestellt ist. Davon unbeschadet hat der mindestens eine Spülmitteldurchgang eine Erstreckungskomponente in Richtung der Längsachse, das heißt, "diagonal" bezieht sich nur auf einen Grundriss, bevorzugt auf einen rotationssymmetrischen, kreisförmigen Grundriss.

Die Richtung der Erstreckung in Längsachse ist dadurch definiert, dass der mindestens eine Spülmitteldurchgang einen Abschnitt aufweist, der eine Erstreckungskomponente parallel zur Längsachse in Richtung des ersten Endes des Elektrodenkörpers aufweist und so die erste Öffnung an der Elektrodenaußenfläche in der Nähe oder an der ersten Anbringungsstelle bildet, so dass der Spülmittelstrom in Richtung der ersten Anbringungsstelle gelenkt wird und so die Elektrode und der erste Katheterschaft gekühlt wird. Der Winkel zwischen dem genannten Abschnitt des Spülmitteldurchganges und der Längsachse beträgt im Gradmaß zwischen 1 und 80 Grad, vorzugsweise zwischen 30 und 60 Grad. Dadurch erhält der aus der Öffnung auf der Elektrodenaußenfläche austretende Spülstrom die entscheidende, in Richtung des Katheterschaftes weisende Richtung und kann somit verhindern, dass eine unzulässige Erwärmung der Grenzregion mit Koagulation von Körperflüssigkeiten geschieht. Es wird also gewährleistet, dass das Spülmedium nicht in orthogonaler Richtung zur Längsachse des Elektrodenkörpers austritt.

Bevorzugt befindet sich die Verbindung zwischen dem Spülmittellumen und dem mindestens einen Spülmitteldurchgang auf einer Ebene zwischen dem ersten und einem zweiten, dem ersten Ende abgewandten, Ende bevorzugt auf der halben Strecke zwischen erstem und zweitem Ende.

Gemäß einer Variante der Erfindung ist die Elektrode als Ringelektrode ausgelegt. Das heißt, dass diese Elektrode keine Endelektrode ist und sich im Anschluss vor oder hinter dieser Elektrode weitere Elektroden an einem ersten Katheterschaft befinden können. Gemäß dieser Variante weist der Elektrodenkörper eine zweite Anbringungsstelle für einen zweiten Katheterschaft an einem zweiten, Ende auf, wobei in eine bevorzugten Ausführung - wenn mehrere gekühlte Ringelektroden vorhanden sind - das Spülmittellumen am zweiten Ende des Elektrodenkörpers eine Öffnung bildet, welche in Verbindung mit einem Lumen des zweiten Katheterschaftes steht. An diesem zweiten Schaft kann dann am gegenüber liegenden Ende eine weitere erfindungsgemäße Elektrode angebracht sein, so dass in Bezug auf diese weitere Elektrode der zweite Katheterschaft dieselbe Funktion wie der erste Katheterschaft in Bezug auf die zuerst genannte Elektrode hat. Vorteilhaft können mehrere solcher Elektroden verschiedene Aufgaben wahrnehmen und den Mess- und Therapieerfolg verbessern.

Im Falle einer solchen Variante der Erfindung wird der Spülmitteldurchgang in vorteilhafterweise Weise so ausgebildet sein, dass sich die zweite Öffnung des Spülmitteldurchganges diametral entgegen gesetzt zur ersten Öffnung an der Elektrodenaußenfläche in der Nähe oder an der zweiten Anbringungsstelle befindet. So wird auch die zweite Anbringungsstelle optimaler Kühlung unterzogen.

In allen Ausführungsformen muss der Spülmitteldurchgang nicht entlang einer Achse verlaufen, er kann auch "gebogen" sein. Beispielsweise kann die Verbindung zwischen dem Spülmittellumen und einem ersten Abschnitt des Spülmitteldurchganges durchaus nahezu orthogonal oder in einem flachen Winkel (45° bis 80°) zur Längsachse sein. Der Spülmitteldurchgang verläuft im Anschluss dann jedoch in Richtung der Elektrodenaußenfläche in einem spitzen Winkel von 1 bis 45°, um so die zu erzielende Funktionalität zu erhalten, die Anbringungsstelle zwischen erstem Ende der Elektrode und Katheterschaft zu kühlen.

Gemäß einer weiteren Variante der Erfindung kann die Elektrode als Kopfelektrode ausgebildet sein. In diesem Falle bildet die Elektrode das äußerste, in einen Körper einführbare Ende eines Katheters. Diese Elektrode ist dadurch gekennzeichnet, dass die Elektrode ein dem ersten Ende des Elektrodenkörpers abgewandten zweiten Ende aufweist, wobei das zweite Ende vorzugsweise atraumatisch, besonders bevorzugt kugelförmig, trapetzförmig oder abgerundet geformt ist und wobei das zweite Ende eine Elektrodenaußenfläche als Elektrodenkopffläche bildet. Dies erleichtert die atraumatische Einführung des Katheters in das Körperlumen und kann zusätzlich die versehentliche Perforation des zu behandelnden Körpergewebes wie beispielsweise das Endokard verhindern.

In dieser Variante der Elektrode sind die zweiten Öffnungen der Spülmitteldurchgänge in der Elektrodenkopffläche gebildet. Dadurch wird bewirkt, dass die auch die "Spitze" des Katheters, welche durch das kugelförmige und damit atraumatische Ende der Elektrode gebildet wird und in der Regel wesentlich für die punktförmige Verödung des Körpergewebes ist, ebenfalls so gekühlt wird, dass keine gesundheitsgefährdende Schädigung des Gewebes und Koagulationen erfolgen.

Die Erfindung betrifft in einem weiteren Aspekt einen elektrophysiologischen Ablationskatheter, der einen lang gestreckten ersten Katheterschaft mit einem proximalen und einem distalen Ende, eine am distalen Ende des Katheterschaftes angebrachte und soeben beschriebene Elektrode, mindestens ein im lang gestreckten Katheterschaft befindliches Lumen, welches sich vom proximalen zum distalen Ende des Katheterschaftes erstreckt, von denen mindestens eines an seinem distalen Ende mit dem Spülmittellumen der genannten erfindungsgemäßen Elektrode in Verbindung steht und an seinem proximalen Ende mit einem Anschluss zur Zuführung des Spülmittels in Verbindung steht, und mindestens eine elektrische Signalleitung zur Übertragung von hochfrequenten Signalen und/oder zur Messung von physiologischen Signalen an soeben beschriebenen Elektrode. Diese Signalleitung verläuft ebenfalls vom proximalen Ende des Katheters, an dem sich eine Verbindung zur Weiterleitung der Messsignale oder zur Einspeisung von hochfrequenten Signalen befindet. Die Leitung kann in einem der genannten Lumen verlaufen, beispielsweise zur Sicherstellung der Kühlung der Signalleitung beispielsweise unter Einfluss elektromagnetischer Strahlung. Eine solche Lösung ist in der US 7,507,237 gezeigt, welche in vollem Umfang in diese Anmeldung einbezogen ist. Die Signalleitung kann alternativ aber auch im Schaftmaterial eingebettet sein.

Optional, aber nicht zwingend für jede Ausführung kann der elektrophysiologische Ablationskatheter Steuermittel umfassen, wobei sich die Steuermittel im und am proximalen Ende des Katheterschaftes befinden. Diese Steuermittel sind wie weiter oben erwähnt Zugdrähte oder Zugseile, welche distal am distalen Ende des Katheterschaftes oder an der erfindungsgemäßen Elektrode befestigt sind und proximal mittels eines bekannten Steuerhandgriffes gesteuert werden können, um so eine Biegung des distalen Bereiches des elektrophysiologischen Katheters zu bewirken.

Weiterhin kann ein solcher Katheter in oder an der Elektrode einen oder mehrere verschiedene Sensoren umfassen, beispielsweise Temperatursensor zur Messung der Temperatur während der Behandlung oder Drucksensoren zur Messung des Anpressdruckes des Katheters an das Gewebe. Die Messsignale dieser Sensoren können wie oben genannt bevorzugt in der Signalleitung zum proximalen Ende geleitet werden oder aber in eigenen Messleitungen.

Bevorzugt umfasst ein erfindungsgemäßer Katheter sowohl Ring- als auch eine Kopfelektrode auf.

Weitere nicht genannte Einzelheiten der Erfindung sind in der Beschreibung genannt.

Es zeigen:
- Fig. 1A: eine Außenansicht einer ersten aus dem Stand der Technik bekannten Ausführung einer Kopfelektrode mit Innenraum und Spülöffnungen
- Fig. 1B: einen Längsschnitt einer ersten aus dem Stand der Technik bekannten Ausführung einer Kopfelektrode mit Innenraum und Spülöffnungen
- Fig. 2A: zeigt die Außenansicht einer weiteren aus dem Stand der Technik bekannten Kopfelektrode mit Durchgängen zu den Spülöffnungen
- Fig. 2B: einen Längsschnitt einer weiteren aus dem Stand der Technik bekannten Kopfelektrode mit Durchgängen zu den Spülöffnungen
- Fig. 3A: eine Außenansicht einer erfindungsgemäßen Kopfelektrode
- Fig. 3B: einen Längsschnitt einer erfindungsgemäßen Kopfelektrode
- Fig. 4A: eine Außenansicht einer erfindungsgemäßen Kopfelektrode, die in den Schaft des ersten Katheteres eingelassen ist
- Fig. 4B: einen Längsschnitt einer erfindungsgemäßen Kopfelektrode, die in den Schaft des ersten Katheteres eingelassen ist
- Fig. 5A: eine Außenansicht einer erfindungsgemäßen Ringelektrode
- Fig. 5B: einen Längsschnitt einer erfindungsgemäßen Ringelektrode

Die Erfindung wird im Folgenden anhand eines Ausführungsbeispieles für eine Kopfelektrode beschrieben. Selbstverständlich kann sich die Erfindung auch auf jegliche Elektrodenform beziehen, wie beispielsweise Ringelektroden.

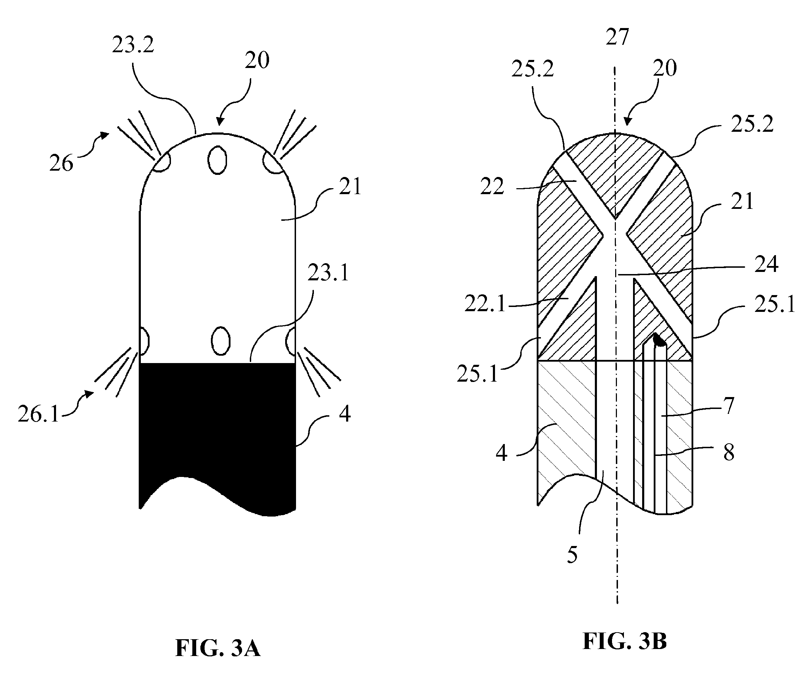

Fig. 3A und Fig. 3B zeigen schematisch eine erste Ausführungsform der Kopfelektrode für einen elektrophysiologischen Katheter mit gleichem Schaftaufbau wie oben beschrieben. Die Kopfelektrode 20 ist durch einen Elektrodenkörper 21 massiv aus Metall gebildet, in welches diametrale Spülmitteldurchgänge 22, 22.1 vorgesehen sind. Die Kopfelektrode 20 hat ein erstes, in diesem Fall proximales Ende 23A, an dem sich eine Anbringungsstelle zur Befestigung an den Katheterschaft 4 befindet. Das Spülmittel (z.B. physiologische Kochsalzlösung) wird über ein Lumen 5 im Katheterschaft 4 durch ein Spülmittellumen 24 in den Kopf geführt, füllt die Durchgänge 22, 22.1 und tritt durch die Öffnungen 25 aus. Ein Teil 26.1 des Spülstroms 26 ist zum proximalen Ende der Kopfelektrode 20, d.h. zur Anbringungsstelle an den Katheterschaft 4 hin gerichtet. Dies wird dadurch bewirkt, dass ein Abschnitt 22.1 des Spülmitteldurchgangs 22 eine Erstreckungskomponente parallel der Längsachse 27 in Richtung des ersten Endes 23.1 des Elektrodenkörpers aufweist und so eine Öffnung 25.1 an der Elektrodenaußenfläche in der Nähe oder an der ersten Anbringungsstelle bildet. In einem weiteren Lumen 7 ist die elektrische Zuleitung 8 zur Kopfelektrode 20 geführt. Nicht gezeigt ist ein optionaler Temperatursensor, welcher im gleichen oder in einem weiteren Lumen geführt sein kann.
Das zweite Ende 23.2, in diesem Falle das distale Ende, ist kugelförmig geformt und die Austrittsöffnungen 25.2 in dieser Elektrodenkopffläche bewirken, dass der Spülstrom auch an dieser Stelle zu einer Kühlung beiträgt.

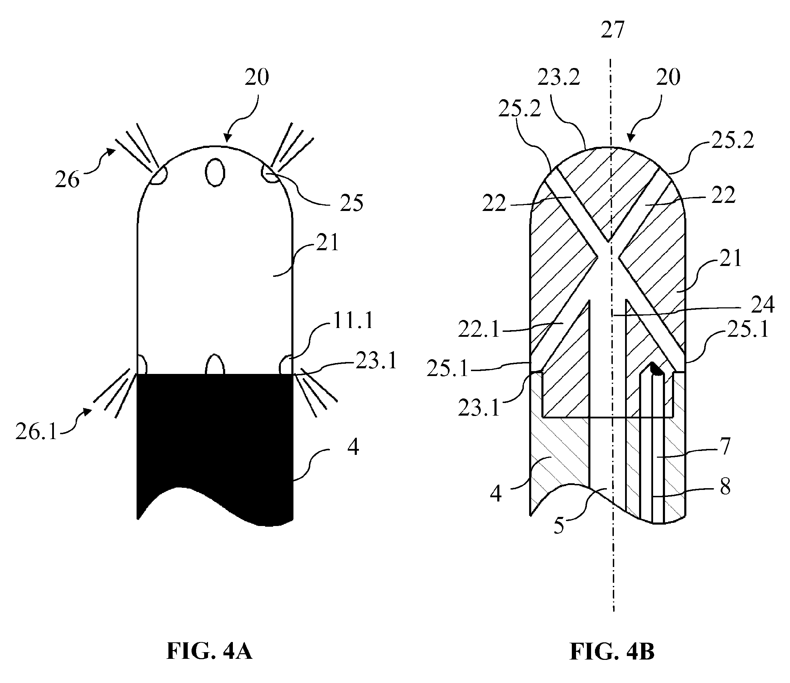

Fig. 4A und Fig. 4B zeigen schematisch eine zweite Ausführungsform der Kopfelektrode für einen elektrophysiologischen Katheter mit gleichem Schaftaufbau wie oben beschrieben. Gleiche oder ähnliche Teile sind mit an Fig. 3A und Fig. 3B angelehnten Bezugsziffern bezeichnet und werden hier nicht nochmals erläutert. Die Kopfelektrode 20 ist so in das distale Ende des Katheterschaftschlauchs 4 eingelassen, dass sich ein Teil 25.1 der Öffnungen 25 der Spülmitteldurchgänge 22, 22.1 genau an der Anbringungsstelle am ersten Ende 23.1 des Elektrodenkörpers befindet, also direkt an der Außenfläche der Kopfelektrode 10 zur Außenfläche des Katheterschaftschlauch befindet, der denselben Außendurchmesser wie die Elektrode aufweist.

In einer verbesserten Variante dieser Ausführungsform kann der Teil 25.1 der Öffnungen 25 am Kopfelektroden/Schaft-Übergang verbreitert sein oder mit einer am Übergang umlaufenden Nut verbunden sein, um die Spülflüssigkeit optimal am Übergang zu verteilen.

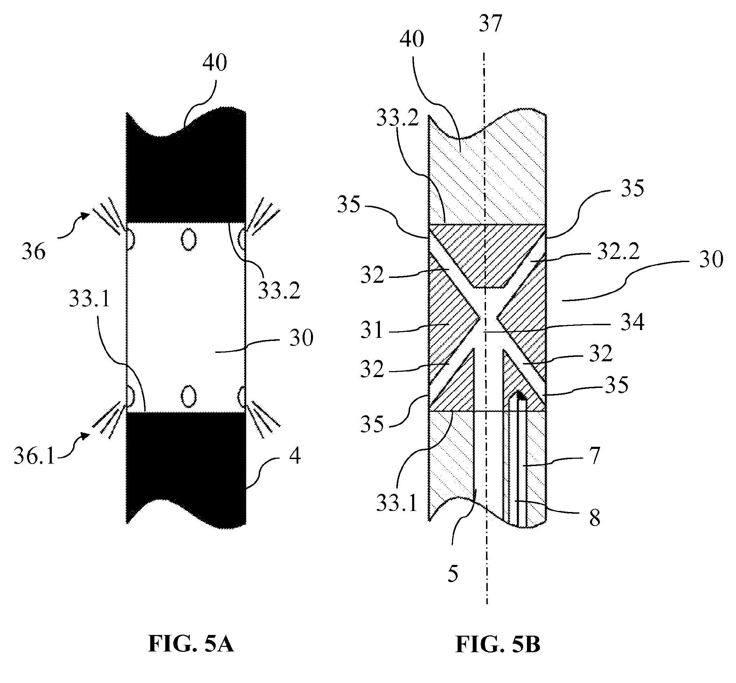

Fig. 5A und Fig. 5B zeigen schematisch eine erste Ausführungsform einer Ringelektrode für einen elektrophysiologischen Katheter mit gleichem Schaftaufbau wie oben beschrieben. Gleiche oder ähnliche Teile sind mit an Fig. 3A, Fig. 3B, Fig. 4A und Fig. 4B angelehnten Bezugsziffern bezeichnet und werden hier nicht nochmals erläutert. So zeigen beispielsweise die Spülmitteldurchgänge 32 dieselben Merkmale wie die Spülmitteldurchgänge 22 und die Öffnungen 35 dieselben Eigenschaften wie die Öffnungen 25 der Kopfelektrode gemäß der Fig. 3A, Fig. 3B, Fig. 4A und Fig. 4B. Ebenso haben Lumen 5 und 7 sowie Zuleitung 8 dieselben Funktionen.
Die Ringelektrode weist in diesem Fall am zweiten Ende 33.2 eine zweite Anbringungsstelle für einen zweiten Katheterschaft 40 auf. Die Öffnungen 35, die die Spülmitteldurchgänge 32 mit der Elektrodenaußenfläche bildet, liegen in dieser Ausführung alle auf der Elektrodenaußenfläche und dienen dazu, die Anbringungsstelle an den Enden 33.1 und 33.2 zu kühlen, um somit Koagulationen zu vermeiden.

In einer verbesserten Variante dieser Ausführungsform können die Öffnungen 35 an den ersten und zweiten Anbringungsstellen verbreitert sein oder mit an den Übergängen umlaufenden Nuten verbunden sein, um die Spülflüssigkeit optimal an den Übergängen zu verteilen.

Gemäß einer weiteren nicht dargestellten Ausführung weist der zweite Katheterschaft 40 dieselben Merkmale bezüglich Lumen 5 und 7 sowie Zuleitung 8 auf. Dazu sind die Zuleitung 8 innerhalb des Lumens 7 isoliert durch die Elektrode geführt, während das Lumen 5 so zum zweiten Ende 33.2 des Elektrodenkörpers weitergeführt wird, dass es eine zweite Öffnung bildet, welche mit dem nicht bezeichneten Lumen in Katheterschaft 40 in Verbindung steht. Dies ermöglicht die Anbringung mehrerer gekühlter Ringelektroden in Reihe.

## Patentansprüche

1. Elektrode für einen elektrophysiologischen Ablationskatheter mit einem sich entlang einer Längsachse erstreckenden Elektrodenkörper, umfassend:
- eine Elektrodenaußenfläche zur Abgabe von hochfrequenten Signalen und/oder zur Messung von physiologischen Signalen;
- eine erste Anbringungsstelle an einem ersten Ende, an welchem die Elektrode an einem ersten Katheterschaft angebracht ist, durch welchen Signale und Spülmittel zur Elektrode geleitet werden können;
- ein Spülmittellumen in Erstreckung parallel zur Längsachse, durch welches Spülmittel aus dem ersten Katheterschaft in die Elektrode geleitet werden kann welches am ersten Ende des Elektrodenkörpers eine Öffnung bildet, welche in Verbindung mit einem Lumen des ersten Katheterschaftes steht; und
- mindestens ein in Verbindung mit dem Spülmittellumen stehender Spülmitteldurchgang, der in einem Winkel zur Längsachse angeordnet ist, wobei der Spülmitteldurchgang an der Elektrodenaußenfläche mindestens eine Öffnung bildet, durch die das Spülmittel in die Umgebung als Spülmittelstrom abgegeben werden kann;
**dadurch gekennzeichnet, dass**
der Winkel ein solches Gradmaß aufweist, dass der Spülmitteldurchgang eine Erstreckungskomponente parallel zur Längsachse der Elektrode aufweist, so dass der aus der mindestens einen Öffnungen austretende Spülmittelstrom eine Erstreckungskomponente entlang der Elektrodenaußenfläche aufweist und sich so verteilt, dass die Elektrode und deren unmittelbarer Umgebung gekühlt ist.

2. Elektrode nach Anspruch 1, **dadurch gekennzeichnet, dass** der mindestens eine Spülmitteldurchgang den Elektrodenkörper diagonal so durchläuft, dass das Spülmittellumen und der mindestens eine Spülmitteldurchgang in Verbindung stehen.

3. Elektrode nach einem der Ansprüche 1 oder 2, **dadurch gekennzeichnet, dass** ein Abschnitt des Spülmitteldurchgangs eine Erstreckungskomponente parallel der Längsachse in Richtung des ersten Endes des Elektrodenkörpers aufweist und so eine erste Öffnung an der Elektrodenaußenfläche in der Nähe oder an der ersten Anbringungsstelle bildet, so dass der Spülmittelstrom in Richtung der ersten Anbringungsstelle abgelenkt wird und so die Elektrode und den ersten Katheterschaft kühlt.

4. Elektrode nach Anspruch 3, **dadurch gekennzeichnet, dass** der Abschnitt des Spülmitteldurchgangs, der eine Erstreckungskomponente parallel der Längsachse in Richtung des ersten Endes des Elektrodenkörpers aufweist zusammen mit der Längsachse einen Winkel im Gradmaß zwischen 1 und 80 Grad, bevorzugt zwischen 30 und 60 Grad umschließt.

5. Elektrode nach einem der Ansprüche 1 bis 4, **dadurch gekennzeichnet, dass** der Spülmitteldurchganges an der diametral entgegen gesetzt zur ersten Öffnung befindlichen Elektrodenaußenfläche eine zweite Öffnung aufweist.

6. Elektrode nach einem der Ansprüche 1 bis 5, **dadurch gekennzeichnet, dass** die Elektrodenaußenfläche im Bereich der ersten und/oder zweiten Öffnung trichterförmige Einbuchtungen oder eine radial umlaufende Nut aufweist, um die Verteilung des Spülmediums entlang der Anbringungsstelle zum ersten Katheterschaft zu gewährleisten.

7. Elektrode nach einem der Ansprüche 1 bis 6, **dadurch gekennzeichnet, dass** der Elektrodenkörper ein zweites, dem ersten Ende abgewandtes, Ende aufweist und sich die Verbindung zwischen dem Spülmittellumen und dem mindestens einen Spülmitteldurchgang auf einer Ebene zwischen dem ersten und zweiten Ende, bevorzugt auf der halben Strecke zwischen erstem und zweitem Ende befindet.

8. Elektrode nach einem der Ansprüche 1 bis 7, **dadurch gekennzeichnet, dass** der Elektrodenkörper eine zweite Anbringungsstelle für einen zweiten Katheterschaft an einem zweiten Ende aufweist, wobei bevorzugt das Spülmittellumen am zweiten Ende des Elektrodenkörpers eine Öffnung bildet, welche in Verbindung mit einem Lumen des zweiten Katheterschaftes steht.

9. Elektrode nach Anspruch 8, **dadurch gekennzeichnet, dass** sich die zweite Öffnung des Spülmitteldurchganges diametral entgegen gesetzt zur ersten Öffnung an der Elektrodenaußenfläche in der Nähe oder an der zweiten Anbringungsstelle befindet.

10. Elektrode nach einem der Ansprüche 1 bis 7, **dadurch gekennzeichnet, dass** die Elektrode eine Kopfelektrode ist mit einem dem ersten Ende des Elektrodenkörpers abgewandten zweiten Ende, wobei das zweite Ende vorzugsweise atraumatisch, besonders bevorzugt halbkugelförmig, trapezförmig oder abgerundet geformt ist und wobei das zweite Ende eine Elektrodenaußenfläche als Elektrodenkopffläche bildet.

11. Elektrode nach Anspruch 10, **dadurch gekennzeichnet, dass** die zweite Öffnung des Spülmitteldurchganges in der Elektrodenkopffläche gebildet ist.

12. Elektrophysiologischer Ablationskatheter, umfassend:
- einen lang gestreckten ersten Katheterschaft mit einem proximalen und einem distalen Ende;
- eine am distalen Ende des Katheterschaftes angebrachte Elektrode nach einem der Ansprüche 1 bis 11;
- mindestens ein im lang gestreckten Katheterschaft befindliches Lumen, welches sich vom proximalen zum distalen Ende erstreckt, wobei mindestens eines dieser Lumen an seinem distalen Ende mit dem Spülmittellumen der Elektrode nach Anspruch 1 bis 11 und an seinem proximalen Ende mit einem Anschluss zur Zuführung des Spülmittels in Verbindung steht;
- mindestens eine elektrische Signalleitung zur Übertragung von hochfrequenten Signalen und/oder zur Messung von physiologischen Signalen an der Elektrode nach einem der Ansprüche 1 bis 11, welche vom proximalen Ende des Katheterschaftes zur Elektrode verläuft.

13. Elektrophysiologischer Ablationskatheter nach Anspruch 12, **dadurch gekennzeichnet, dass** der Katheter mindestens eine Ringelektrode nach einem der Ansprüche 8 oder 9 und eine Kopfelektrode nach einem der Ansprüche 10 oder 11 umfasst.
